**Europäisches Patentamt**

(19) **European Patent Office**

**Office européen des brevets**

(11) Publication number: **0 158 690**

**A1**

(12) # EUROPEAN PATENT APPLICATION

(21) Application number: **84104365.6**

(22) Date of filing: **17.04.84**

(51) Int. Cl.⁴: **G 01 R 29/08**

(43) Date of publication of application:
23.10.85 Bulletin 85/43

(84) Designated Contracting States:
AT BE CH DE FR GB IT LI LU NL SE

(71) Applicant: Gillespie, Anthony Richard
45 Wood Park
Ivybridge Devon PL21 0PP(GB)

(71) Applicant: Haslam, Christopher Glyn Trevor
Regerstrasse 20
D-5308 Rheinbach(DE)

(71) Applicant: Haslam, Nicola Catherine
24 Sandringham Drive
Wistaston Crewe CW2 8JF(GB)

(72) Inventor: Gillespie, Anthony Richard
45 Wood Park
Ivybridge Devon PL21 0PP(GB)

(72) Inventor: Haslam, Christopher Glyn Trevor
Regerstrasse 20
D-5308 Rheinbach(DE)

(72) Inventor: Haslam, Nicola Catherine
24 Sandringham Drive
Wistaston Crewe CW2 8JF(GB)

(74) Representative: Dipl.-Phys.Dr. Manitz Dipl.-Ing.,
Dipl.-Wirtsch. Finsterwald Dipl.-Chem.Dr. Heyn
Dipl.-Phys. Rotermund Morgan, B.Sc.(Phys)
Robert-Koch-Strasse 1
D-8000 München 22(DE)

(54) **Thermographic apparatus for measuring the temperature distribution in a substantially dielectric medium.**

(57) A thermographic apparatus is provided for measuring the temperature distribution in a substantially dielectric medium by detecting the microwave energy emerging from the medium. The apparatus is particularly useful for detecting carcinomas of the human body. The apparatus comprises an array of microwave antennas positioned adjacent the patient's body and electronic means for processing the broad band signals induced in the microwave antennas to determine the temperature prevailing in adjacent volume elements of the patient's body. The electronic means is laid out to process the signals induced in each of the antennas and to correlate each signal with the signal from one of the antennas in a fixed reference location. The correlation produces first and second values containing information relating to the amplitude and phase of the signal received in the or each detecting position of that antenna relative to the amplitude and phase of the signal received by the reference antenna. A computer is used to carry out an inverse transformation of the relative amplitude and phase signals from all the antennas to produce a matrix of temperature values relating to adjacent volumes of the patient's body.

EP 0 158 690 A1

### Thermographic Apparatus for Measuring the Temperature Distribution in a Substantially Dielectric Medium

The present invention relates to thermographic apparatus for measuring the temperature distribution in a substantially dielectric medium by detecting the microwave energy emerging from the medium, and has particular reference to thermographic apparatus for use in medical applications. Such medical applications include the detection of carcinomas or lesions of the human body which manifest themselves by a locally elevated temperature and increase in emission of black-body radiation. In this case the human body forms the substantially dielectric medium. The thermographic apparatus can however also be used to determine temperature distributions in other dielectric media.

So far as the medical applications are concerned it is known that the region surrounding a carcinoma usually shows an excess of temperature over the mean temperature of the human body, and that this effect is extended to a larger region by the flow of blood from the excess of blood vessels found around the carcinomas. This means that one can in practice detect the carcinomas with a local temperature excess of only $1.5^\circ$ K using thermographic

apparatus. As the temperature distribution is measured solely by detecting the microwave energy emerging from the patient's body the techniques of thermography are passive and non-invasive and can thus be used for the routine screening of people for cancer or other infections without any danger of side effects. Existing thermographic apparatus has been successfully used for the detection of carcinomas, in particular carcinomas of the female breasts, it is not however really suitable for mass screening because of the large time required to take the necessary measurements.

Typical prior art thermographic apparatus and methods are described in the articles "Detection of Breast Cancer by Microwave Radiometry" by A.H. Barrett, P.C. Myers in the journal "Radio Science 12" (supplement) 1977, and "Centimeter- and Millimeter-Wave Thermography - A survey on Tumor Detection" by J. Edrich in "The Journal of Microwave Power" vol. 13, No. 2, 1979, pages 95 to 104. In the first mentioned article a rectangular waveguide antenna filled with a low loss solid with a dielectric constant equal to 11 is placed flush against the patient's skin and a plurality of readings are taken at different locations, typically nine different locations on each breast. In each position the antenna receives signals from a substantial volume of the patent's body and a relatively high reading indicates that a carcinoma may be present in that particular volume of the patient's body. The carcinoma can be more precisely localised by comparing the readings from adjacent positions of the antenna. It is possible, although not discussed by these authors, to use an array of independent antennas to give several simultaneous readings. This would shorten the time taken for an examination, but would not give any increase in spatial resolution. In the method described in the second article the patient is laid on a table and a receiving arrangement, comprising a reflector and a horn antenna mounted in front of the reflector, is mounted above the patient and is movable within a cartesian coordinate system. In this way the microwave radiation emerging from the patient's body is directed by the reflector to the horn antenna and different areas of the body can be scanned by moving the receiver arrangement to different coordinate positions. Again a plurality of readings have to be taken and this takes a substantial amount of time.

Another method of thermography is described in the article "New Correlation Radiometer for Microwave Thermography" by A. Mamouni, J.C. van de Velde and Y. Leroy in Electronic Letters August 6th, 1981, vol. 17, No. 16. This article describes a receiver which does not consist of a single probe (or antenna) as in the usual microwave radiometers, but consists of a combination of two or several probes adjacent to each other and in contact with the patient's body. The idea underlying this arrangement is that each probe will receive microwave signals from an adjacent volume of tissue and that two adjacent volumes of tissue associated with two adjacent probes will have a region of overlap common to both probes. The electronic circuitry associated with the two probes is said to be such that the contribution of the thermal noise generated in the common volume of overlap can be identified. It is stated that a delay introduced into one of the two signal paths from the two probes can be used to modify the contribution of the different sub-volumes in the volume of overlap. The problem with this arrangement however is that it is only intended to produce signals from the regions of overlap between adjacent probes which will be small, giving better resolution than with single probes, but temperature mapping of a whole area of a body is again only possible by producing relative movement between the probes and the body. Thus again a plurality of readings is necessary, which takes an undesirably long time. Furthermore, the spatial resolution of this and other prior art thermographic apparatus are relatively restricted.

The principal object underlying the present invention is
to provide thermographic apparatus which is able to
give an image of a whole area of a dielectric medium, in
particular of a patient's body, and to measure the temperature distribution in the medium with substantially improved
spatial and thermal resolution in a short time.

In order to satisfy this object there is provided, in
accordance with the invention, and starting from the prior
art apparatus described in the article in Electronics
Letters as cited above, thermographic apparatus for
measuring the temperature distribution in a substantially
dielectric medium by detecting the microwave energy
emerging from the medium, the apparatus comprising an
array of microwave antennas positioned adjacent the dielectric medium and electronic means for processing the broad-
band signals induced in the microwave antennas in a
plurality of detecting positions to determine the temperature prevailing in a volume element of the dielectric
medium, characterised in that the electronic means com-
prises means for processing the signal induced in each
of the antennas in each of the detecting positions and for
correlating each signal with the signal from at least one
of said antennas in a fixed reference location to produce,
for the or each detecting position of each antenna, first
and second values containing information relating to the
amplitude and phase of the signal received in that detecting position relative to the amplitude and phase of the
signal received by at least one of the reference antennas;
and computing means for forming an inverse transformation of
the first and second values associated with each antenna of
the array, said inverse transformation being a matrix of
temperature values relating to adjacent volumes of said
dielectric medium.

This apparatus is thus based on the principle of aperture
synthesis as used in radio astronomy from which it is known that the distribution of amplitude and phase over an antenna's aperture is the Fourier transform of the source's brightness distribution. This is a significant extension of correlation techniques to give an image of the brightness distribution in the whole of the region of response of the individual antennas, which are made so that a large region of the patient's body is in each and every antenna's response pattern. Using the apparatus of the invention it should be possible to measure the temperature distribution over a whole region of a patient's body of approximately the same size as the array size within a very short space of time and with high spatial and thermal resolution. The short period of time required for the measurement not only makes the apparatus suitable for mass screening techniques, but also for monitoring the effects of treatments to the patient on virtually any time scale. Since the signals are correlated across the extremities of the array, the apparatus will have the high resolution appropriate to the array size over the whole region examined. It is believed that the presently proposed apparatus will be capable of detecting deep-seated hot spots $1.5^{\circ}$ K above ambient temperature with a positional accuracy of a few millimeters in a short time.

The inverse transformation of the first and second values is preferably an inverse Fourier transformation. The inverse Fourier transformation is however theoretically only valid for temperature sources which are at a substantial distance from the antennas. If a straightforward inverse Fourier transformation produces results which are obviously inconsistent it may be necessary to make certain corrections, or to resort to an inverse Fresnel transformation, or even to make corrections and use an inverse Fresnel transformation.

In view of this, one embodiment of the apparatus is characterised by the provision of phase adjustment means for adjusting the relative phase of each signal induced in each of the antennas in the or each detecting position, relative to the phase of the signal received by at least one of the reference antennas, prior to effecting the inverse transformation, whereby to compensate for near field effects. Thus this embodiment envisages that the corrections required to the inverse Fourier transformation, or possibly to the inverse Fresnel transformation can be made by correcting the relative phases of the signals prior to effecting the inverse transformation. This adjustment of the relative phases is most effectively made by software embodied in the computing means which will enable phase corrections to be effected and the output to be analysed. If at all possible it is preferable to use an inverse Fourier transformation rather than an inverse Fresnel transformation because the calculations involved in performing an inverse Fourier transformation are substantially easier.

A particularly preferred development of the apparatus is characterised in that an inert dielectric layer of high permittivity, preferably greater than 10, and of low loss, preferably better than 5 %, is placed between said array and said medium. The inert dielectric layer is preferably greater than 5 cm thick and should preferably be contoured so that it at least approximately fits the shape of the patient's body, in order to avoid a skin/air interface which would result in substantial and undesirable losses. In this way the array of antennas can also be spaced further away from the patient without significant loss in signal strength. Because the array is spaced further from the patient the inverse transformation may be simplified so that it is straightforwardly possible to use an inverse Fourier transformation.

In a further development of the apparatus each of the antennas may be replaced by two or more antenna elements, whereby separate polarisation components can be measured either prior to or after the inverse transformation, and means may be provided for compensating for polarisation differences.

This embodiment recognises that it may be essential to take account of the state of polarisation of the signals received by the individual antennas.

In a particularly preferred embodiment means are provided for varying the centre frequency and/or the bandwidth at which the measurement of the temperature distribution is carried out.

This embodiment is based on the recognition that the correct choice of frequency and/or bandwidth can yield more information on the temperature distribution inside the patient's body. In particular, the taking of several measurements at different centre frequencies may be necessary for a depth dimension to be added to the measured temperature distribution. It will be appreciated that the measured temperature distribution may be regarded as a two-dimensional projected temperature distribution, similar to an X-ray which is a two-dimensional projected density distribution. A theoretical discussion of the points to be considered when selecting frequency and bandwidth is included at the end of this specification.

In one practical embodiment for varying said centre frequency said electronic means comprises a local oscillator and a mixer for generating a difference frequency, with the local oscillator frequency being variable whereby to vary said centre frequency. This embodiment may include amplifier and/or filter means for varying said bandwidth.

In an alternative arrangement the electronic means does not include a local oscillator but is characterised in that the centre frequency and bandwidth are both determined by an amplifier and/or a filter.

In a further practical embodiment the array of microwave antennas is spatially fixed, so that each antenna has one detecting position, and switching means is provided for connecting said antennas groupwise in turn to said electronic means. In this way it is possible to provide a large number of antennas in the array without having to provide a separate electronic channel for each individual antenna, which would be very costly. Instead selected groups of antennas are connected in turn to a like number of electronic channels.

Alternatively, a smaller number of antennas is arranged in an array and means can be provided for moving the array so that each antenna, other than the reference antenna or antennas, adopts or moves through a plurality of detecting positions. In this embodiment a respective electronic channel is associated with each antenna, however the total number of antennas is substantially reduced.

The movable array can be a linear array, which may be rotated or moved laterally, and which is relatively simple to construct, it can however also comprise a two-dimensional array of non-uniformly spaced antennas. This latter arrangement is particularly advantageous because the layout of the individual antennas in the array can be selected : so that cross-talk between the individual antennas is avoided.

Said plurality of detecting positions preferably fill the area over which said array is moved. This embodiment recognises that the amount of information which can be gathered is greatest if, in the course of taking the measurements, a measurement is made at each element of the area over which the said array is moved.

In a further development of the apparatus intermediate storage means is provided for temporarily storing said signals from each of said antennas in the or each detecting position. This embodiment makes it possible to temporarily store the signals so that they may be subsequently fed to the computing means in the correct time sequence.

In a particularly preferred embodiment the electronic means includes a noise calibration system with a noise generating source, such as a noise diode, adapted to feed noise to each of said antennas and said calibration system is optionally adapted to calibrate the apparatus to take account of reflections at the surface of the medium and/or at boundaries within the medium.

The noise calibration system enables drift in the electronics to be recognised and makes it possible for suitable corrective measures to be taken. In a further development automatic gain control means are provided, being optionally controlled in dependence on a signal derived from the said noise calibration system.

In a particularly preferred embodiment the electronic means comprises first multiplier means for forming the products of the signal from at least one of the reference antennas with the signal induced in each of the remaining antennas in the or each detecting position to yield said first values; phase shifting means for shifting the phase of the signal induced in each of the remaining antennas in the or each detecting position by $90^{\circ}$ to produce phase shifted signals; and second multiplying means for forming the products of the signal from the same reference antenna with each of the phase shifted signals to yield said second values. When using this embodiment the first and second values have the general form of the product of an amplitude term and the sine and cosine of a phase difference term respectively. The phase and amplitude of the signal received in each antenna in the or each detecting position relative to the signal received by the reference antenna can be directly calculated from the first and second values.

The antennas preferably comprise dipole antennas or antennas formed as printed circuits on dielectric material as all of the region in the patient being examined must be in the primary beam of each antenna at all times during the examination. Such antennas have the advantage of being both inexpensive and effective and a second, crossed dipole can be easily added to each dipole antenna for polarisation measurements.

Finally, the present invention recognises that existing hyperthermia apparatus, i.e. apparatus used to treat carcinomas by heating them to elevated temperatures, could be dramatically improved by combining it with the presently proposed thermographic apparatus. Such combined apparatus will be characterised by means for operating the thermographic apparatus and the hyperthermia apparatus in alternative time periods, optionally using the same antennas, whereby to detect the temperature distribution produced by the hyperthermia apparatus and, optionally, by means for controlling the hyperthermia apparatus using the signals from the thermographic apparatus.

Because the thermographic apparatus of the present invention should produce, for the first time, a very rapid read-out of the temperature distribution within the patient's body it should be possible to energise the hyperthermia apparatus for a short while, to measure the actual temperature distribution produced using the termographic apparatus, to correct the relative phases and amplitudes of the microwave signals fed to the antennas of the hyperthermia apparatus, to carry out further heating to re-measure the temperature distribution, and to repeat this process so as to ultimately produce a desired temperature distribution in the body using the hyperthermia apparatus.

In other words the apparatus of the present invention could be used to provide a real time image of the temperature distribution around a site being subjected to hyperthermia treatment, with the image scanning process being time-shared with the application of hyperthermia power. With this arrangement it will of course be necessary to provide adequate thermal protection for the receiver preamplifier stages. If the time sharing intervals are small, say 0.1. seconds, the cooling effects of the bio-heat transport mechanisms around the area will be negligible, thus the thermogram represents the true temperature distribution during the hyperthermia process. This should greatly aid the preferential destruction of the cancers and the implementation of high resolution phased array hyperthermia systems. The apparatus of the present invention should give a high enough resolution to match the size of area warmed by the hyperthermia treatment.

A further technique which might be useful is to shine a narrow band noise source thorugh the body. The power required for an adequate signal is quite small (1 mW) and does not represent a health hazard. This signal would be blinked and detected synchronously in the computer software. The method may reveal variations in the opacity of the tissue as a function of frequency which can be detected by image comparison and may be found in clinical practice to correlate with abnormalities.

Embodiments of the invention will now be described in further detail by way of example only and with reference to the accompanying drawings which show:

Fig. 1  a table adapted to support a patient during a
        thermographic investigation,

Fig. 2  a plan view of a fixed square antenna array,

Fig. 3a a plan view of rotatable linear antenna array,

Fig. 3b a plan view of a laterally movable linear antenna
         array,

Fig. 4   a schematic section on the line IV-IV of the array
         of Fig. 3a illustrating the position of a hot spot
         which is to be detected,

Fig. 5   a schematic block diagram of the arrangement of
         the electronic circuitry used to process the
         signals from the antennas of the array of Figs. 3
         and 4,

Fig. 6   a schematic block diagram illustrating in more
         detail the construction of the correlation blocks
         shown in the block diagram of Fig. 5,

Fig. 7   a diagram illustrating the noise calibration
         principle underlying the circuitry of Figs. 5 and 6,
         and

Fig. 8   the temperature difference seen just into a dielectric for:
         a) an isolated hot spot of diameter 1 cm with
            $T = 1.5^\circ$ K above normal body temperature as seen
            through various thicknesses of muscles,
         b) a layer of warmer material seen under 2 mm of
            skin and 1 cm of fat for various thicknesses of
            muscle for air ( $\varepsilon_r = 1$) and dielectric
            ( $\varepsilon_r = 30$). In both cases the material has the
            electrical properties of muscle.

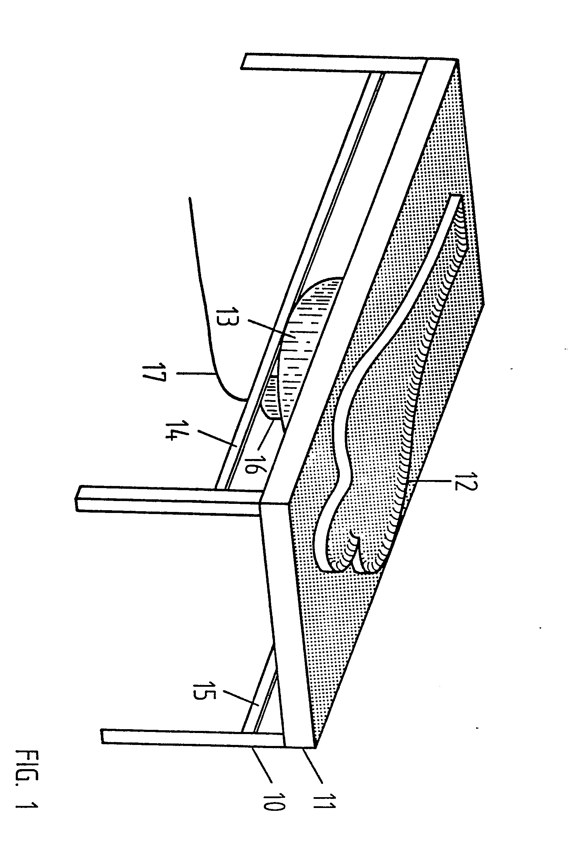

Turning firstly to Fig. 1 there can be seen a table 10
supporting a slab of dielectric 11 of high relative
permittivity $\varepsilon_r$ = 30 and of low loss (less than 5%). A
material of this kind is sold under the trademark "HiK" by
Emerson and Cuming, Canton, Massachusetts 02021, USA. The
upper surface of the slab of dielectric is provided with moulded contours 12 which correspond approximately to the shape of the human body. Alternatively, slabs of moulded material could be slidably arranged on the basic slab of dielectric 11 so that they could be moved to provide a best fit for any particular patient. An antenna array 13 is positioned immediately beneath the slab of dielectric 11 in contact therewith and can be slid to and fro beneath the table on a pair of horizontal rails 14, 15, so that it can be positioned under the part of the patient's body under investigation.

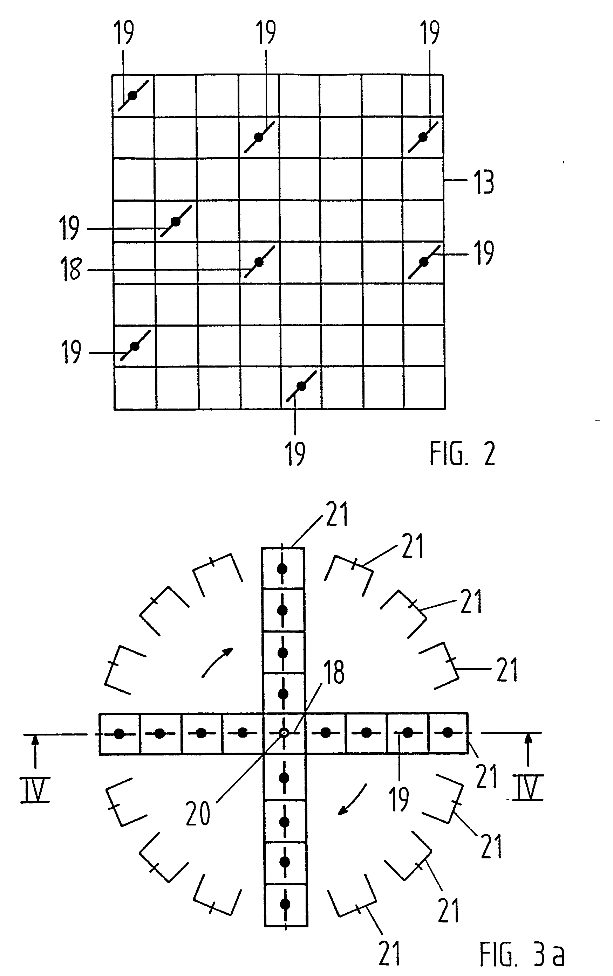

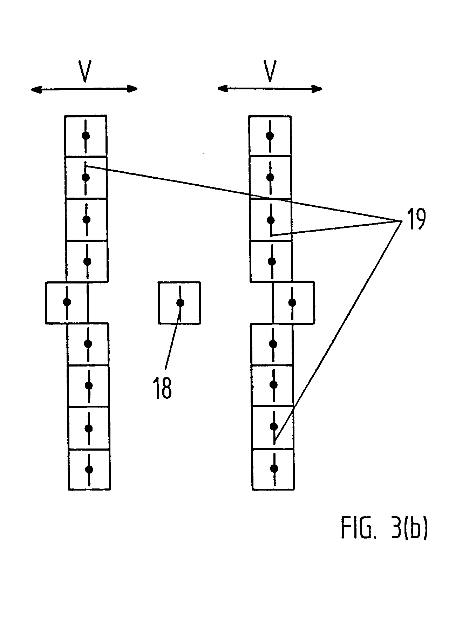

The antenna array shown in Fig. 1 is in fact a rotatable linear antenna array as shown in Fig. 3a) and is rotated by a motor 16 disposed centrally beneath the array. The drive leads for the motor and the connections between the electronics mounted on the antenna array and the further electronics are schematically illustrated by the reference numeral 17. The antenna array may alternatively be a fixed antenna array such as the square antenna array 13' shown in Fig. 2.

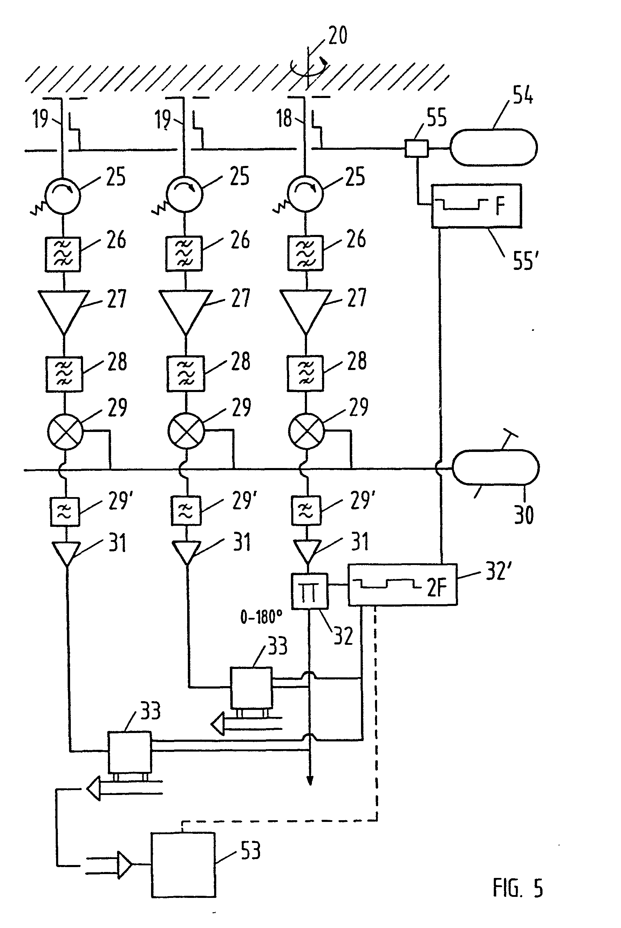

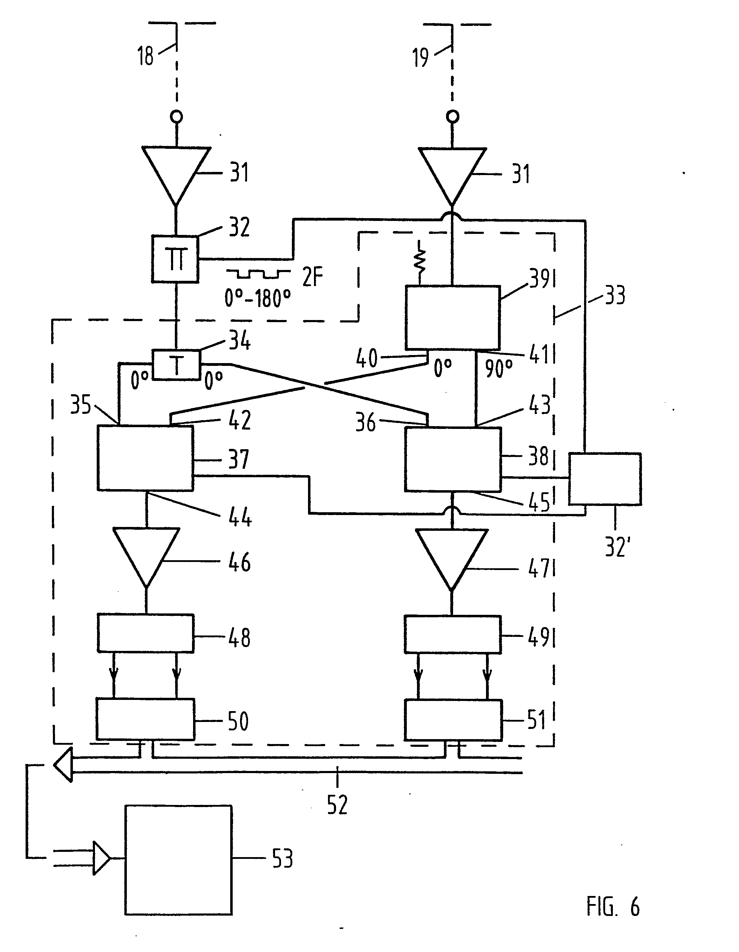

In the square antenna array of Fig. 2 there are eight rows and eight columns of antennas 18,19 each in the form of a dipole antenna with one of the antennas 18 forming a reference antenna. Although it would be theoretically possible to derive signals from each of said antennas simultaneously this would in fact require 64 processing channels which would probably be prohibitively expensive. For this reason it is preferred to provide say eight channels and to use electronic switching techniques to switch between selected groups of eight antennas. One of the antennas, the reference antenna 18, must of course always be connected to one of the channels, the reference channel, because the existence of a reference antenna is crucial to the measurement process as will later become clear from the discussions of Figs. 5 and 6.

The groups of antennas which are connected to the eight channels at any time are preferably spread across the surface of the array so that cross-talk between individual antennas is minimised. This is illustrated in Fig. 2 which only shows antennas in eight distinct fields of the array, namely the antenna 18, which is the reference antenna, and the further antennas 19 which constitute one of the groups of antennas which are connected to the remaining seven electronic channels. It will be understood that identical antennas are provided in each field of the array, these antennas have however been omitted in order to simplify the illustration.

Rather than providing such a large number of individual antennas it is also possible to provide fewer antennas and to move the array to different detection positions as illustrated in Fig. 3a + b. In this arrangement it is however essential that one antenna, in this case the antenna 18, should be fixedly positioned for comparison purposes. The linear array of Fig. 3 is rotatable about the central axis 20 through 180$^{\circ}$ through a plurality of distinct detecting positions 21. This can be effected by the motor 16 of Fig. 1 which can be either a stepping motor or a continuously rotating motor. I.e. readings can be taken while the array is stationary or when it is moving.

An alternative arrangement is shown in Fig. 3b in which two linear arrays are positioned on either side of a central fixed reference antenna 18 and can be displaced linearly to the left and to the right relative to the central antenna as illustrated by the arrows V. This embodiment has the advantage that the antennas do not rotate and thus respond to the same polarisation components during the course of the examination.

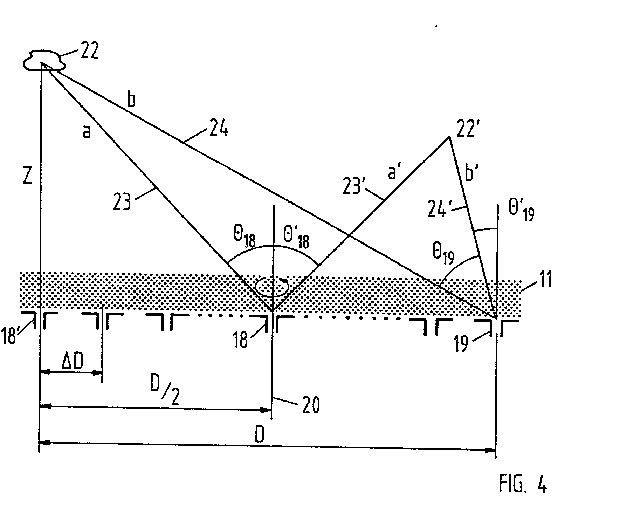

The array of Fig. 3a is shown again in cross-section in Fig. 4 which also shows the slab of dielectric 11 and a hot spot 22 which is assumed to lie somewhere in the patient's body.

Fig. 4 also shows radiation emitted from one point in the hot spot which propagates in the general direction of the lines 23 and 24 and is detected by the two antennas 18 and 19. It can easily be seen that the signal induced in the antenna 18 will have a different phase from that of the signal induced in the antenna 19. Of course the antenna 18 and the antenna 19 are not just receiving radiation from the hot spot 22 but also from every other point in the patient's body such as the point 22'.

The antenna's beam pattern (its response to radiation from angles $\Theta$ off its principal axis) must be broad enough to ensure that it covers all the area to be examined. That is to say that the whole of the area to be examined should be in the primary beam of every antenna at all times.

It can be seen from Fig. 4 that the paths 23 and 24 will be mainly in the lossy medium of the patient's body when the hot spot 22 is at the edge of the area examined. This may degrade the signal-to-noise ratio so an optional solution is to take another antenna 18' and use that as a secondary reference antenna. The output from this antenna must also be combined with the output from the other antennas in the manner described below to give secondary desired first and second values. These secondary values can then, in principle, be eliminated during processing prior to the inverse transformation. The use of this technique does not change the principles underlying the following discussion.

The signals generated at the antennas 18 and 19 from the radiation emerging from the hot spot 22 may be described by formulae of the form:

$$V_{18} = V_{22}L_{23}\; f(\theta_{18})\; \cos\left(\omega t - \frac{2\pi a}{\lambda}\right) \qquad\qquad \text{(A)}$$

$$V_{19} = V_{22}L_{24}\; f(\theta_{19})\; \cos\left(\omega t - \frac{2\pi b}{\lambda}\right) \qquad\qquad \text{(B)}$$

where $V_{22}$ is the signal leaving the point 22; $L_{23}$ and $L_{24}$ are the losses along the paths 23 and 24 of Fig. 4 respectively; $f(\theta)$ is the beam pattern of the individual antennas (assumed to be the same for all antennas), with the angles $\theta_{18}$, $\theta_{19}$ being defined as shown in Fig. 4; and $\omega$ is the angular frequency of the radiation leaving the point 22; a and b are the lengths of the paths 23 and 24 respectively, and $\lambda$ is the wavelength in the dielectric.

The expressions for the signals arising at the antennas 18 and 19 from the point 24 are similar vis:

$$V'_{18} = V'_{22}L_{23'}\; f(\theta'_{18})\; \cos\left(\omega t - \frac{2\pi a'}{\lambda}\right) \qquad\qquad \text{(C)}$$

$$V'_{19} = V'_{22}L_{24'}\; f(\theta'_{19})\; \cos\left(\omega t - \frac{2\pi b'}{\lambda}\right) \qquad\qquad \text{(D)}$$

and indeed similar equations can be written for the energy arriving at the two antennas from each point in the patient's body.

On summing these signals one obtains

$$\langle V_{18}\rangle = \iint V(\theta,a).L(\theta,a).f(\theta).\cos\left(\omega t - \frac{2\pi a}{\lambda}\right).d\theta da \qquad \text{(E)}$$

$$\langle V_{19}\rangle = \iint V(\theta,b).L(\theta,b).f(\theta).\cos\left(\omega t - \frac{2\pi b}{\lambda}\right).d\theta db \qquad \text{(F)}$$

These can be expressed in the form $\langle V_{18} \rangle = \tilde{V}_{18} \cos (\omega t - \Psi_{18})$ and $\langle V_{19} \rangle = \tilde{V}_{19} \cos(\omega t - \Psi_{19})$ where $\tilde{V}_{18}$ and $\tilde{V}_{19}$ are mean voltages and $\Psi_{18}$ and $\Psi_{19}$ are mean phase shifts and these signals now have to be processed and correlated to obtain the amplitude and phase of the net signal received at antenna 19 relative to the signal received at antenna 18. This processing is done by the electronic circuitry shown in Figs. 5 and 6.

Referring firstly to Fig. 5 it can be seen that the signals received by each of the antennas is directed through a respective channel. Each channel first contains a circulator 25 which is a device for ensuring that energy flows primarily from the antenna to the detection channel and not vice versa. Each circulator 25 is followed by a bandpass filter 26 which may be incorporated in the preamplifiers 27.

Thereafter the signals in each channel are amplified in preamplifiers 27 and are passed to image rejecting circuits 28 which reduce the noise present in the mixer image band, coming from the broadband preamplifiers. These filters reduce the uncorrelated noise present in the signals thus improving the signal-to-noise ratio. At this stage the signals are fed into respective single-sideband mixers 29 and are mixed with a signal supplied by a local oscillator 30, which is common to all electronic channels. The difference frequencies produced by the mixers 29 are then fed to low pass filters 29' and then to the respective frequency amplifiers 31. The signal in the reference channel associated with the reference antenna 18 is then passed through a phase switching device 32 which will be described later. Thereafter the signal from the reference channel is passed to the correlators 33 provided in the remaining channels. One of these correlators is illustrated in detail in Fig. 6.

As can be seen the signal received by the correlator 33 of Fig. 6 from the reference channel is first split at a T-splitter 34 into two signals which are passed to respective inputs 35 and 36 of first and second linear multipliers 37, 38 with a dynamic range greater than 40 dB.

The signal coming from the respectively associated antenna 19 is passed to a broad band $90^{\circ}$ splitter 39 (bandwidth 500 mHz on a 3 GHz system) which has two outputs 40 and 41. The signal at the output 40 has not been phase-shifted and is fed to a second input 42 of the first multiplier 37. The second output 41 has a phase shift of $90^{\circ}$ relative to the input signal and is passed to the second input 43 of the second linear multiplier 38. The signals received at the inputs 35 and 42 have the general form

$$V_{35} = \tilde{V}_{18} \cos(\omega t - \Psi_{18}) \qquad (G)$$

$$V_{42} = \tilde{V}_{19} \cos(\omega t - \Psi_{19}) \qquad (H)$$

and the signals received at the inputs 36 and 43 have the general form

$$V_{36} = \tilde{V}_{18} \cos(\omega t - \Psi_{18}) \qquad (I)$$

$$V_{43} = \tilde{V}_{19} \cos(\omega t - \Psi_{19} + \frac{\pi}{2}) \qquad (J)$$

The signals appearing at the outputs 44 and 45 of the first and second linear multipliers 37 and 38 respectively have the general form:

$$S_{44} = \overline{V_{18}V_{19}} \cos(\Psi_{18} - \Psi_{19}) \qquad (K)$$

$$S_{45} = \overline{V_{18}V_{19}} \sin(\Psi_{18} - \Psi_{19}) \qquad (L)$$

The bar denotes the time average of the product of the signals $\tilde{V}_{18}$ and $\tilde{V}_{19}$.

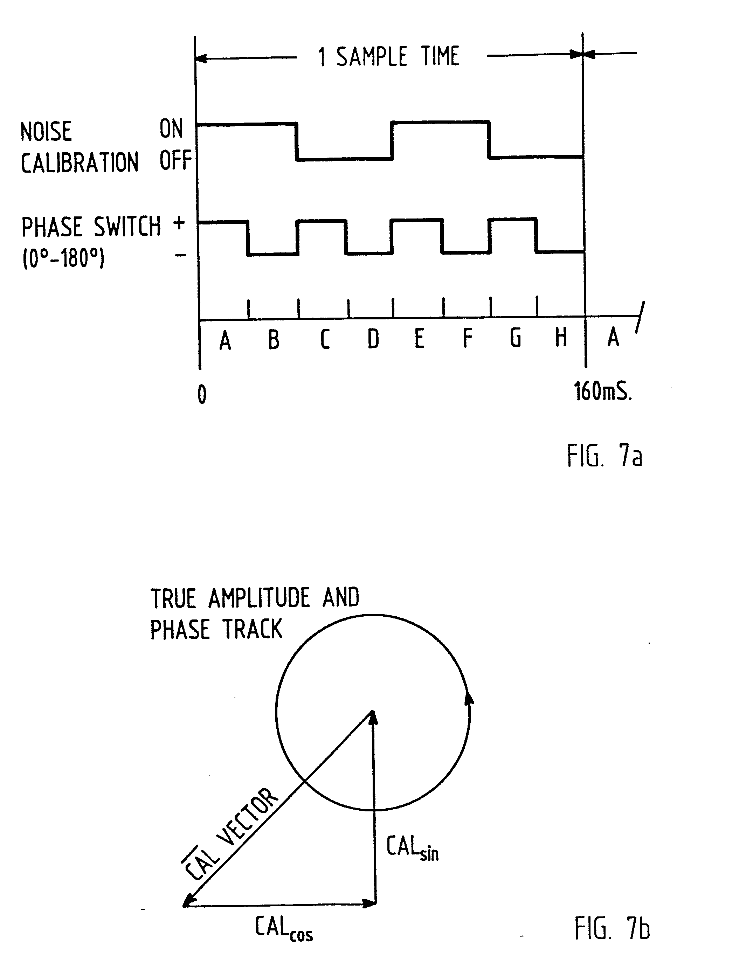

These signals are now integrated by converting them via respective voltage to frequency convertors 46, 47 into frequency signals which are then integrated in 16 bit counters 48, 49 and fed onto a 16 bit databus via respective buffer stores 50, 51. A cycle of 8 such 20 msec samples labelled A - H is given in the example shown in Fig. 7. Each sample represents the correlated power received during the sample period. The sign of the correlation is reversed for each alternate sample by the phase switch 32, shown in Figs. 5 and 6. The samples are combined by subtracting in pairs thus (A-B), (C-D), (E-F), (G-H) for the example given. This procedure effectively corrects for zero drifts in the multipliers 37, 38 and the voltage to frequency convertors 46, 47. The cosine correlation is thus formed using elements 37, 46, 48 and 50 to form $A_c$, $B_c$, $C_c$, $D_c$, $E_c$, $F_c$, $G_c$, $H_c$ and elements 38, 47, 49 and 51 to form $A_s$, $B_s$, $C_s$, $D_s$, $E_s$, $F_s$, $G_s$, $H_s$. These are fed by the 16 bit databus 52 into a microprocessor which forms the computing means and which is adapted to manipulate the first and second values received from the buffer stores 50, 51 via the databus to calculate the relative phase and amplitude at each antenna 19 relative to the reference antenna 18. The relative phase and amplitude are calculated from the equations

$$\text{Cosine} = (A_c - B_c) + (C_c - D_c) + (E_c - F_c) + (G_c - G_c) = S_{44'} \qquad (M)$$

$$\text{Sine} = (A_s - B_s) + (C_s - D_s) + (E_s - F_s) + (G_s - G_s) = S_{45'} \qquad (N)$$

$$\text{Then relative amplitude} = A = \sqrt{S_{44'}^2 + S_{45'}^2} \qquad (O)$$

$$\text{relative phase} = \bar{\phi} = \tan^{-1}(S_{45'}/S_{44'}) \qquad (P)$$

These are computed for each correlated antenna pair.

The microprocessor is then programmed to calculate the inverse Fourier transform from the relative phase and amplitude values for each of the antennas in each of the detection positions, and to produce the output as a matrix of temperature values relating to adjacent volumes of said dielectric medium. This matrix of temperature values can of course be displayed in various ways, for example on the screen of a monitor, possibly with different colours being ascribed to different temperature values, and can also be stored on a floppy disk for future reference or for comparison with images made during successive examinations.

If, as previously-mentioned, it is found that the Fourier transformation is not satisfactory because the array is too close to the patient then it is possible to change the relative phases calculated from the first and second values by calculated or previously empirically derived amounts and to improve the final result of the inverse transformation.

Alternatively it is possible to calibrate the apparatus by using a suitable body of dielectric with known high temperature hot spots therein. The phase corrections required to bring the measured distribution calculated by the inverse Fourier transformation into agreement with the actual temperature distribution can be recorded and subsequently used to correct the relative phases calculated when using the apparatus on a patient.

So far as carrying out the inverse Fourier transformation is concerned the microprocessor is programmed to carry out the following calculation:

$$f(l,m) = \iint g(x,y) \exp \left\{ 2\pi i(xl+ym) \right\} dxdy$$

In this expression 1 and m are the coordinates of a plane close to the patient's body and the function $f(s,m)$ is the desired matrix of temperature values. For any given value of 1 and m, $f(l,m)$ gives the value of the temperature contribution through the body projected onto this plane. x and y are the coordinates, expressed in wavelengths, of the plane containing the antenna array. The origin of this coordinate system, i.e. $x = 0$, $y = 0$ is preferably the reference antenna and the origin of the plane close to the patient's body, i.e. $l=0$, $m=0$ will be directly over this. The function $g(x,y)$ is complex and is the measured distribution across the plane of the array. As an example for the positions in Fig. 4 and taking antenna 18 as the reference antenna, the measured value for $g(x,y)$ will be $g(x_{19},y_{19}) = S_{44}, +i\ S_{45}$, where $i = \sqrt{-1}$.

When the patient is close to the array a Fourier transform will not give adequate results. In this case close to means less than the "far field" criterion given by $z = 2\ D^2/\lambda$ using the notation of Fig. 4. When z is only slightly less than $2\ D^2/\lambda$ it will be possible to obtain adequate results by putting in a phase correction to the Fourier transform, but for smaller values of z the patient is in the near field or Fresnel region. In this case the microprocessor has to be programmed to carry out an inverse Fresnel transform:

$$f(l,m) = \iint \exp\left\{i(xl + ym)\right\}\ g(x,y)\ \exp\left\{-\ i(a_x x^2 + a_y y^2)\right\}\ dxdy$$

In this equation $a_x$ and $a_y$ are parameters of the form: $a_n = k_n \pi/r_n \lambda$ where r is the distance from the reference antenna to the point in the l,m plane and $r_n$ is the length of the projection of r on the appropriate axis. $k_n$ is a parameter which will depend on the phase shifts along the path $a_n$. The other symbols have their previous meanings. This equation gives the transform for a two dimensional picture of the temperature distribution, analogous to an x-ray picture. It may be possible in practice to obtain some estimate of the temperature distribution in the z direction, in which case the equation will need to be modified appropriately.

One of the problems which can arise with thermographic apparatus is the problem of drift which can seriously affect the results. In order to overcome this drift, or at least to recognise it so that its effects may be taken into account, the apparatus may be provided with automatic gain control circuits which may be incorporated as a modification to the amplifiers 31. The present apparatus is provided with a continous noise calibration system as a preferred alternative solution.

This system consists essentially of the noise diode 54 illustrated in Fig. 5 which feeds a small noise signal into each of the antennas 18, 19. A circuit 55 is provided for modulating the output of the noise diode 54 with a square wave at a frequency F. The result of this is equivalent to turning the noise diode on and off at the frequency F, the noise diode should however not actually be switched on and off as this would make it unstable.

The phase switch 32 present only in the electronic channel for the reference antenna serves to periodically change the phase of the signal in the reference channel by 180$^\circ$ and at a frequncy 2F which must be as integer multiple or vulgar fraction of F. The phase switch 32 is driven by a phase switch oscillator 32' which is linked synchronously to the noise switch oscillator 55' and the correlators 33, or alternatively (as shown in broken lines) to the noise switch oscillator 55' and the computer 53. This makes phase sensitive detection possible.

The effect of the noise calibration and of the phase switch is illustrated by an example shown in Fig. 7a over one sample period of 160 msec. It will be noted that this period of 160 msec is subdivided into eight portions each of length 1/4F. As has been described earlier the circuit of Fig. 6 performs the correlated integral for each of the 20 msec portions A - H and all eight values from each multiplier are passed onto the databus. The microprocessor, in addition to computing the sine and cosine correlation terms using the formula (A-B)+(C-D)+(E-F)+(G-H) computes also the signal contributed by the noise diode 54 in Fig. 5 and which is demodulated using the formula (A-B)--(C-D)+(E-F)-(G-H). This signal in a period of 160 msec has a significant measurement error due to noise but this can be reduced to a sufficiently accurate level by fitting a linear least squares solution to the noise diode calibration readings over a period of a minute or so. This signal can also be used to control the gain of the appropriate channel (automatic gain control AGC). In any case it is used in the software to provide a normalisation calibration factor so that changes in receiver gain can be compensated for in the analysis. As is shown in Fig. 7b this noise calibration vector must be subtracted from the outputs of the cosine and  sine channel readings to get true amplitude and phase information.

When the reflection correction technique is used the noise diode is used to inject noise into the antenna lines so that this signal is radiated out into the dielectric media (at a very low level). This corrects for reflection losses at dielectric boundaries. The amplitude and phase of the reflected signal can be demodulated in the same way as the gain calibration signal, and has also to be subtracted from the sine and cosine channels to get the true readings. This noise reflection correction technique has been described in U.S. Patent 4,235,107 for single antenna measurements.

As mentioned earlier it will probably be necessary to take account of the state of polarisation of the signals received in each channel. Polarisation components can be defined and measured in many ways but all methods of determining  them in a synthesis system rely on antenna elements at each antenna position that each respond to one or both polarisation components. The signal from one element (e.g. 18 in Fig. 6) is then combined with the signal from either its "double" or its "complement" at the other antenna position (19, Fig. 6). The other pair then requires another set of electronics (Figs. 5+6). Full polarisation data is then obtained by either adding or subtracting the outputs of the two pairs either in hardware or in software before or after the transformation. The combination chosen is a design parameter. It may however also be possible to make a combined antenna that responds to more than one type of polarisation giving an output proportional to the total signal arriving at that point.

In order to provide good contact between the patient and the table it may be desirable to use a high dielectric constant cushion which may be capable of being moulded to the patient's body shape or a thin polythene envelope of warm water. In either case the intention is to avoid a patient air interface which would give rise to undesirable losses.

Finally, the following comments are made concerning the system parameters and components.

a) Spatial Resolution

Resolution is used here in the sense of the full beam width given by $\theta = \lambda/D$. For an array at a distance z from the object being examined the resolution is then $r = \lambda z/D$ cm, expressed in terms of distance in the patient (see Fig. 4). D/z can only have a value between 0.5 and 5 for practical reasons, with 4 being a reasonable value, but the wavelength can be reduced by a factor of 5.5, using a dielectric with $\varepsilon_r = 30$ between the patient and the array giving a corresponding improvement in resolution. This gives a resolution of

$$r = \frac{cz}{\nu D \sqrt{\mathcal{E}_r}} \qquad (Q)$$

where c is the velocity of height, and $\nu$ is the observing frequency = $\omega/2\pi$ .

r is given in Table 1 (page 33 ) for several frequencies with D/z = 4. The use of a dielectric has two further advantages: (i) the small antennae can be mounted on a rotating piece of similar dielectric enabling strip line techniques to be used in their construction, and perhaps also in the local oscillator supply, and signal amplifier and mixer chains; (ii) suitable shaping of the material to give good contact to the patient's body-shape will considerably improve the matching of the body to the antennae as there is then no longer a skin to air interface (high to low $\mathcal{E}_r$).

The resolution given above, which is of the order of 0.5 to 2.5 cm for 3 GHz > $\nu$ > 1 GHz, is <u>not</u> the error in the determination of the position of the hotter region, as beam-fitting techniques can give the position of small sources to within 0.1 beam widths even when the signal-to-noise ratio of the signal is only 5:1. In this context beam fitting means numerical techniques which give a best fit of a diffraction pattern to a small part of the matrix of temperature values.

b) Operating Frequency

The choice of frequency must consider four factors affecting the visibility of a hot region of fixed size inside the body:

(i)     the radiation's penetration (1/e) depth in tissue increases with wavelength, the absorption coefficient is given approximately by the following empirically derived formula:

$$\alpha = 2.2 \times 10^{-5} \sqrt{\nu} + 0.1 \qquad\qquad (R)$$

over the frequency range 100 MHz to 3 GHz;

(ii)    the hot spot's optical depth increases with frequency giving an increase in effective temperature rise above the surroundings;

Optical depth ($\tau$) is a measure of the absorption of radiation of a particular wavelength as it passes through a lossy medium. For a piece of this medium, the radiation intensity emerging is $e^{-\tau}$ times the incident radiation intensity. For black- body radiation from the lossy medium,which we consider here, the intensity arising from it will be $(1 - e^{-\tau})T$ where T is the temperature of the medium;

(iii)   when the hot-spot's diameter is less than the diameter of the antenna arrays synthesised beam at that point the hot-spot's measured temperature is reduced by the ratio of the beam diamter to hot- spot diameter;

(iv)    the reflections at interfaces inside the body between materials of different permittivities cause losses.

Quantifying effects i, ii and iii, and using equations (A) and (B), lead to a relation for the change in temperature seen at the skin's surface

$$\Delta T_e = \Delta T\, \mathcal{E}_r \left(\frac{D\,k\,\nu}{zc}\right)^2 \left[1 - \exp\left\{- (2.2 \times 10^{-5}\sqrt{\nu} + 0.1)\, k\right\}\right]$$

$$\cdot \exp\left\{- (2.2 \times 10^{-5}\sqrt{\nu} + 0.1)\, b\right\} \qquad (S)$$

where: $\Delta T$ is the hot spot's actual temperature rise above its surroundings, $= 1.5\,K$ in this example,

k is the diameter of the hot spot assumed to be a uniform disc of thickness k, $= 1\,cm$ in this example,

b is the depth of the hot spot below the body surface.

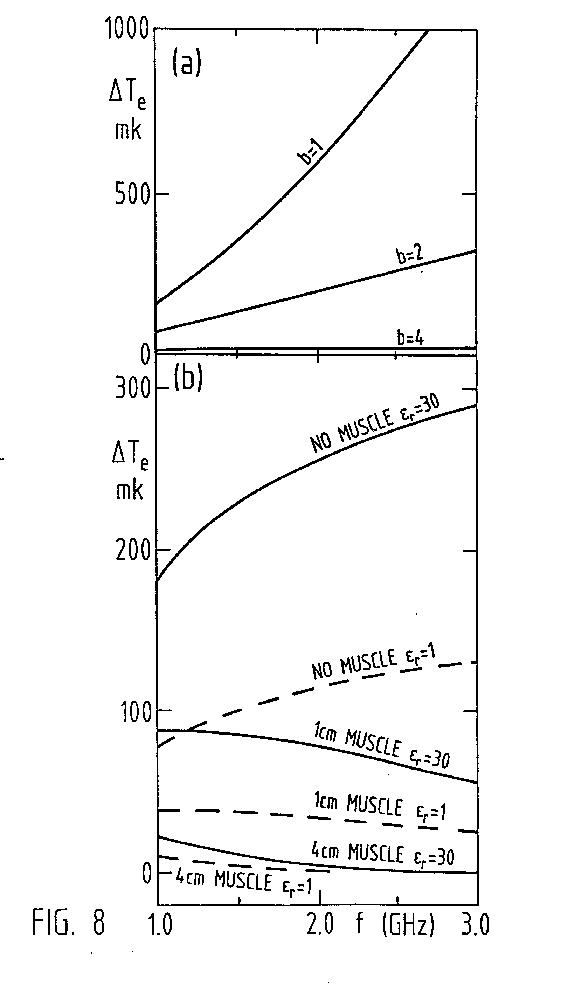

The value of $\Delta T_e$ for several parameters is shown in Fig.8a. Fat has been ignored here because its absorption coefficient is much lower than above, but shows the same variation with frequency. The effect of mismatches at boundaries due to effect (iv) above can be seen in Fig. 8b which gives the temperature measured just outside the skin for a 1.5 cm thick layer of hotter material seen through 2 mm of skin and layers of muscle and fat. This is based on the following relation which has been mathematically derived from the information given in the paper by J. Edrich in "The Journal Of Microwave Power" vol. 14, No. 2, 1979, pages 95 to 104:

$$\Delta T_e = \Delta T \left[1 - \exp\left(-\frac{z_t}{d_t}\right)\right] \cdot \exp\left(-\frac{z_f}{d_f}\right) \cdot e_{fs} \cdot$$

$$\cdot \exp\left(-\frac{z_s}{d_s}\right) \cdot e_{so} \cdot \exp\left(-\frac{z_m}{d_m}\right) \cdot e_{mf} \qquad (T)$$

where: $z_t,\ z_f,\ z_s,\ z_m$ are the thicknesses of the layers of tumour, fat, skin and muscle respectively,

$d_t,\ d_f,\ d_s,\ d_m$ are the 1/e penetration depths of the materials at a given frequency,

$e_{fs},\ e_{so},\ e_{mf}$ are the emissivities from fat to skin, skin to outside the body, and muscle to fat.

Consideration of Fig. 8 shows that for small hot spots higher frequencies are better, until either $\Delta T_e$ levels off or equation (R) breaks down ($> 3$ GHZ), whilst for larger hot areas the lower frequencies are better. Therefore the choice of operating frequency, within the range 900 to 3000 MHz, must depend on the particular application being considered.

Closely related to the operating frequency is the usable bandwidth. This must be sufficiently small for the phase difference between signals at the antennae with the largest separation, $A_1$ and $A_2$ in Fig. 4, not to change by more than $90^{\circ}$ across the band for points at the edge of the picture area. Taking the picture area to be the same as the antenna array area and directly over it, we come to the relation:

$$\Delta\nu < \frac{\nu^2}{4c}\ \frac{xz}{(x-z)} \tag{U}$$

where $\Delta\nu$ is the bandwidth and $x = z^2 + D^2$. This is a very weak constraint, varying from 11 % to 40 % of the operating frequency over the range 915 MHz to 3000 MHz, and the bandwidths given on page 33 are less than this.

c) The Antenna Array

In the far-field case, the number of antennas is determined by the minimum separation between antennas ($\Delta D$) for the lowest Fourier component to have a radius larger than the picture size. In our case, but with far-field approximations, the restriction is that $\lambda/\Delta D > \phi$ where $\phi$ is the angle subtended by the examined area at one element of the array, with $\phi = \tan^{-1} (D/z)$. Expressed in terms of frequency we have:

$$\Delta D < \frac{c}{\nu} \left[ \sqrt{\varepsilon_r}\, \tan^{-1} \left( \frac{D}{z} \right) \right]^{-1} \tag{V}$$

and the number of antennae.

$$n = \frac{D}{\triangle D} + 1 \qquad (W)$$

For $D/z = 4$, $\triangle D$ is of the order of one or two centimetres so dipoles, with suitable balancing networks, are ideal as the individual antennas. The patient is in the near field of the array so it is not possible to reduce the number of antennas by the usual procedure of having unequally spaced antennas separated by multiples of $\triangle D$ and reconstructing the whole aperture using combinations of these as used in raio astronomy. Although this increases the array costs it is offset to some extent by the fact that the path compensators and phase rotators used in astronomy are unnecessary and the data rate is not too high for a small computer handle.

d) Preamplifiers

Many preamplifiers are necessary as all antenna pairs are present so they must be inexpensive. Since the array looks at a 300 K patient this is the lowest possible overall system noise temperature with a perfect amplifier. With the calibration methods discussed in the specification directional coupler, circulator and filter are needed between the dipoles and preamplifiers giving a small degradation in system noise temperature. A simplified sketch of the system is shown in Fig. 5. For our calculations we have assumed a system noise temperature of 600 K = 300 K patient + 300 K amplifier since such amplifiers are readily available at low cost.

e) The Correlators and Continuous Calibration

The amplitude (A) and phase ($\phi$) are measured as sine (S) and cosine (C) terms, where $A^2 = S^2 + C^2$ and $\phi = \tan^{-1}(S/C)$, by cross correlation of the signal from the central receiver with that of a receiver at the appropriate spacing for each of the spacings. In the system proposed the bandwidths are much larger than those used in astronomical synthesis so the correlators must be carefully designed so that the relative phase between inputs is less than a few degrees across the whole bandwidth (200·MHz for the 1.5 GHz system).

In addition they need at least a 40 dB dynamic range (1 mK to 10 K). This specification can be achieved by the use of linear multipliers and by $180^{\circ}$ broad band phase switching in one of the I.F. inputs (see Fig. 6). When synchronous demodulation is applied this removes the 5 % or so square law response inherent in the multipliers at higher signal levels and simultaneously corrects for zero drift in the multipliers and following voltage-to-frequency convertors. These convertors, in conjunction with buffered counters, act as integrators. Gain calibration for each channel is derived by a modulated noise source switched at half the phase inversion rate and which is weakly (-25 dB) but <u>coherently</u> coupled into the input circuit of each preamplifier, see Fig. 5. Software demodulation makes it possible to carry out real time correction, during the analysis, for system drifts of phase and amplitude and provides engineering monitoring of possible system malfunctions.

f) Inverse Transform and Image Matrix

During the rotation of the array we need to collect $D\tilde{w}/\Delta D$ samples each of N-1 amplitudes and phases to match the minimum separation limit calculated in section c. For the 1.5 GHz system this gives 57 samples each of 18 amplitudes and phases. We have chosen 64 samples each lasting 160 milliseconds in the software demodulation example previously described. As the array rotates the amplitude and phase information is interpolated into a matrix of values in the aperture plane and the continuous calibration corrections previously described are computed and applied
at the end of the scan directly into the aperture plane
array. This array is then transformed to form the image.
This transform may well require special purpose hardware
to take full advantage of the short 10 second exposure time
required by the receivers.

The image matrix requires a minimum of two points per
resolution element which for the 1.5 GHz system is 0.91 cm
(Table 1, page 27) giving a grid point separation of 0.45 cm.
A 50 cm square image then needs a matrix of 111 x 111
points. If each element has 2 byte accuracy (1 in 65536) we
can provide a temperature range of 10 K relative to some
conveniently chosen zero for the image with an accuracy of
0.15 mK due to round-off. Such an image would require 25 K
bytes including identification and calibration information
and sixteen such images could be stored on a single
0.5 Megabyte floppy disk including patient case history
details. This would give a convenient cheap filing
method for hospital use, allowing later off-line inspection
by a consultant and eventual transfer to mass storage for
large sample statistical analysis.

The image matrix could well have the format described in
the article "NOD 2 A general system of analysis for
radioastronomy" by C.G.T. Haslam in Astronomy and Astrophysics Supplement vol. 15 p.p. 333-335, 1974 which is
very efficient in computer time when used with two-
dimensional regridding and interpolation routines. These
operations will be essential for comparing pictures taken
on different days, since the patient will not lie in
exactly the same place, so superposition, shifts of scale,
translation and rotation will be required before detailed
comparison can be made. Suitable software and necessary
algorithms, including techniques for combining overlapping images, have been described, in an astronomical
context, in the article "NOD 2 A Generalised System of
Data Analysis for Astronomy which has Application to Image Processing" by C.G.T. Haslam, N.C. Haslam and D.T. Emerson, Technischer Bericht 56 des Max-Planck-Instituts für Radioastronomie Bonn, 1980.

Table 1

System Parameters.

Maximum antenna separation 50 cms.

Distance from patient to antenna array 12.5 cms.

Dielectric constant of table   30

System noise temperature 300 K + 300 K from patient.

| Frequency | 915 | 1500 | 2450 | 3000 | MHz |
|---|---|---|---|---|---|
| Resolution | 1.49 | 0.91 | 0.55 | 0.45 | cms |
| Number of antennae in array | 12 | 19 | 30 | 37 | |
| Bandwidth | 100 | 200 | 400 | 500 | MHz |
| R.m.s. noise in 10 second exposure | 5.47 | 3.07 | 1.73 | 1.39 | milli Kelvin |
| Time taken to see 1.5 K warmer layer below 2 mm skin, 1 cm fat and 4 cm muscle | 6 | 9 | 57 | 222 | seconds |

1. Thermographic apparatus for measuring the temperature distribution in a substantially dielectric medium by detecting the microwave energy emerging from the medium, the apparatus comprising an array (13; 13') of microwave antennas (18, 19) positioned adjacent the dielectric medium and electronic means (Figs. 5 + 6) for processing the broad-band signals induced in the microwave antennas (18, 19) in a plurality of detecting positions (21) to determine the temperature prevailing in a volume element of the dielectric medium, characterised in that the electronic means (Figs. 5 + 6) comprises means (25, 26, 27, 28, 29, 29', 30, 31, 33) for processing the signals induced in each of the antennas (18, 19) in each of the detecting positions (21) and for correlating each signal with the signal from at least one of said antennas (18) in a fixed reference location to produce, for the or each detecting position (21) of each antenna (19), first and second values containing information relating to the amplitude and phase of the signal received in that detecting position relative to the amplitude and phase of the signal received by at least one of the reference antennas (18); and computing means (53) for forming an inverse transormation of the first and second values associated with each antenna of the array, the result of said inverse transformation being a matrix of temperature values relating to adjacent volumes of said dielectric medium.

2. Thermographic apparatus in accordance with claim 1 and characterised by the provision of phase adjustment means for adjusting the phase of each signal induced in each of the antennas (19) in the or each detecting position, relative to the phase of the signal received by at least one of the reference antennas (18), prior to effecting the inverse transformation, whereby to compensate for near field effects.

3. Thermographic apparatus in accordance with either of the preceding claims, characterised in that an inert dielectric layer (11) of high permittivity, preferably greater than 10, and of low loss, preferably better than 5 %, is placed between said array (13, 13') and said medium.

4. Thermographic apparatus in accordance with claim 3, characterised in that said dielectric layer (11) is greater than 5 cms thick.

5. Thermographic apparatus in accordance with any one of the preceding claims, characterised in that each of the antennas (18, 19) is replaced by two or more antenna elements whereby separate polarisation components can be measured either prior to or after the inverse transformation, and, optionally, in that means are provided for compensating for polarisation differences.

6. Thermographic apparatus in accordance with any one of the preceding claims, characterised in that means (29, 29'; 30, 31) are provided for varying the centre frequency and/or the bandwidth at which the measurement of the temperature distribution is carried out.

7. Thermographic apparatus in accordance with claim 6, characterised in that said electronic means (Figs. 5 + 6) comprises a local oscillator (30) and a mixer (29) for generating a difference frequency, with said local oscillator frequency being variable whereby to vary said centre frequency.

8. Thermographic apparatus in accordance with claim 7, characterised in that amplifier (31) and/or filter means is provided for varying said bandwidth.

9.  Thermographic apparatus in accordance with claim 6
    wherein said electronic means does not include a mixer
    and/or local oscillator, characterised in that the centre
    frequency and bandwidth are determined by an amplifier.

10. Thermographic apparatus in accordance with any one of
    the preceding claims, characterised in that said array
    (13') of microwave antennas (18, 19) is spatially fixed,
    whereby each antenna (18, 19) has one detecting
    position; and in that switching means is provided for
    connecting said antennas groupwise in turn to said electronic means (Figs. 5 + 6).

11. Thermographic apparatus in accordance with any one of the
    preceding claims 1 to 9, characterised in that means
    (16) is provided for moving said array (13) so that each
    antenna (19) other than said reference antenna (18) or
    antennas (18') adopts or moves through a plurality of
    detecting positions.

12. Thermographic apparatus in accordance with claim 11,
    characterised in that said movable array comprises one
    or more linear arrays (13).

13. Thermographic apparatus in accordance with claim 11,
    characterised in that said movable array comprises a
    plurality of non-linearly spaced antennas.

14. Thermographic apparatus in accordace with any one of
    the preceding claims, characterised in that intermediate
    storage means      is provided for temporarily storing
    said signals from each of said antennas (18, 19) in the
    or each detecting position.

15. Thermographic apparatus in accordance with any one of
    the preceding claims, characterised in that said electronic means (Figs. 5 + 6) includes a noise calibration system (54, 55, 55') with a noise generating source (54), such as a noise diode, adapted to feed noise to each of said antennas (18, 19); and in that said calibration _ system (54, 55, 55') is optionally adapted to calibrate the apparatus to take account of reflections at the surface of the medium and/or at boundaries within the medium.

16. Thermographic apparatus in accordance with any one of the preceding claims, characterised in that said electronic means (Figs. 5 + 6) further comprises means for controlling the gain of the said electronic means automatically, said automatic gain control being optionally controlled in dependence on a signal derived from the said noise calibration system.

17. Thermographic apparatus in accordance with any one of the preceding claims characterised in that said electronic means (Figs. 5 + 6) comprises first multiplier means (37) for forming the products of the signal from the reference antenna (18) with the signal induced in each of the remaining antennas (19) in the or each detecting position to yield said first values; phase shifting means (39) for shifting the phase of the signal induced in each of the remaining antennas in the or each detecting position by $90^O$ to produce phase shifted signals; and second multiplying means (38) for forming the products of the signal from the reference antenna (18) with each of the phase shifted signals to yield said second values.

18. Thermographic apparatus in accordance with claims 15 and 17 characterised in that said electronic means (Figs. 5 + 6) further comprises means (32) for periodically changing the phase of the signal from the reference antenna by $180^O$ with the change taking place at a first frequency (2F) and optionally further means (55) for periodically switching the output of said noise source (54) between a selected value and zero at a second frequency (F) equivalent to said first frequency (2F) divided by or multiplied by an integer, and means for periodically sampling said first and second values at intervals of time (1/4F) equivalent to one half of the reciprocal of said first frequency ; and in that said computing means (53) is adapted to process said sampled first and second values to derive calibration information and signal information.

19. Thermographic apparatus in accordance with any one of the preceding claims characterised in that said antennas comprise dipole antennas (18, 19) or antennas formed as printed circuits on dielectric material.

20. The combination of thermographic apparatus in accordance with any one of the preceding claims with hyperthermia apparatus characterised by means for operating the thermographic apparatus and the hyperthermia apparatus in alternative time periods, optionally using the same antennas, whereby to detect the temperature distribution produced by the hyperthermia apparatus and, optionally, by means for controlling the hyperthermia apparatus using the signals from the thermographic apparatus.

21. The combination of thermographic apparatus in accordance with any one of the preceding claims with a narrow band noise source located on the side of said dielectric medium opposite to said thermographic apparatus, wherein said narrow band noise source is blinked and detected synchronously  by said computing means.

0158690

FIG. 1

FIG. 2

FIG. 3a

V            V

19

18

FIG. 3(b)

FIG. 4

FIG. 5

FIG. 6

6/8

0158690

FIG. 7a

FIG. 7b

FIG. 8

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A-2 052 909 (INSTRUMENTARIUM CY.) <br> * Page 2, lines 10-40; figure 1 * | 1 | G 01 R 29/08 |
| A | | 3 | |
| | --- | | |
| Y | CONFERENCE PROCEEDINGS, 12th EUROPEAN MICROWAVE CONFERENCE, 13th-17th September 1982, pages 553-558, Helsinki, FI; A. MAMOUNI et al.: "Introduction to correlation microwave thermography" <br> * Page 554, figure 1; paragraph: "Principle of the correlation microwave thermography" * | 1 | |
| | --- | | TECHNICAL FIELDS SEARCHED (Int. Cl.4) |
| Y | US-A-4 178 100 (ROBERT A. FROSCH) <br> * Abstract; figure 2 * | 1 | G 01 R <br> A 61 B <br> A 61 N |
| A | | 6,12, 15,16 | |
| | --- | | |
| A | US-A-4 416 552 (ROBERT A. HESSEMER, LLOYD J. PERPER) <br> * Figures 12-16; claims 1,2, 4-7, 14-15, 18 * | 5-9 | |
| | ---     -/- | | |

The present search report has been drawn up for all claims

| Place of search <br> THE HAGUE | Date of completion of the search <br> 10-12-1984 | Examiner <br> KAUFFMANN J. |
|---|---|---|

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int Cl 4) |
|---|---|---|---|
| A | 1982 IEEE MTT-S INTERNATIONAL MICROWAVE SYMPOSIUM DIGEST, 15th-17th June 1982, session paper V2, pages 438-440, Dallas, USA; F. STERZER et al.: "A self-balancing microwave radiometer for non-invasively measuring the temperature of subcutaneous tissues during localised hyperthermia treatments of canc er" * Figure 2 * | 19,20 | |
| A | IEEE MTT-S INTERNATIONAL MICROWAVE SYMPOSIUM DIGEST 1983, 31st May - 3rd June, Session F4, pages 186-188, Boston, USA; Y. LEROY et al.: "Present results and trends in microwave thermography" | | TECHNICAL FIELDS SEARCHED (Int. Cl.4) |

The present search report has been drawn up for all claims

| Place of search THE HAGUE | Date of completion of the search 10-12-1984 | Examiner KAUFFMANN J. |
|---|---|---|